# EUROPÄISCHE PATENTSCHRIFT

(11) **EP 1 315 711 B1**
(45) Veröffentlichungstag und Bekanntmachung des Hinweises auf die Patenterteilung: **24.03.2004**
(21) Anmeldenummer: 01969620.2
(22) Anmeldetag: 21.08.2001
(51) Int. Cl.: C07D 311/20

(54) **VERFAHREN ZUR HERSTELLUNG VON DIHYDROCUMARIN DURCH HYDRIERUNG VON CUMARIN**
METHOD FOR PRODUCING DIHYDROCOUMARIN BY HYDROGENATING COUMARIN
PROCEDE DE PREPARATION DE DIHYDROCOUMARINE PAR HYDRATATION DE COUMARINE

(30) Priorität: 01.09.2000 DE 10043094
(43) Veröffentlichungstag der Anmeldung: 04.06.2003
(73) Patentinhaber: Symrise GmbH & Co. KG, 37603 Holzminden (DE)
(72) Erfinder: KUHN, Walter, 37603 Holzminden (DE); FUNK, Hans-Ulrich, 37697 Lauenförde (DE); SENFT, Gerhard, 37603 Holzminden (DE)
(74) Vertreter: Eisenführ, Speiser & Partner
(86) Internationale Anmeldenummer: PCT/EP2001/009605
(87) Internationale Veröffentlichungsnummer: WO 2002/018360

(56) Entgegenhaltungen:
- EP-A- 0 420 532
- US-A- 5 237 075

## Beschreibung

Die Erfindung betrifft ein Verfahren zur Herstellung von Dihydrocumarin durch Hydrierung von Cumarin in Gegenwart eines Palladiumkatalysators.

In J. Am. Chem. Soc., 62, 1940, 283-287 ist die Hydrierung von Cumarin zu Dihydrocumarin unter Raney-Nickel-Katalyse bei 100°C beschrieben. Die Hydrierung wird in Diethylether bei einem Druck von 100 bar durchgeführt und ergibt nach Destillation Dihydrocumarin in 90 % Ausbeute.

In US 3,442,910 wird die Herstellung von Dihydrocumarin und Derivaten beschrieben, indem 2-Oxocyclohexanpropionsäure in Gegenwart von Platin, Palladium, Nickel, Rhodium oder Ruthenium-Katalysator dehydriert und cyclisiert wird. Bei dieser Reaktion werden Temperaturen von 275 bis 325°C benötigt. Als Lösungsmittel wird bevorzugt Diphenylether eingesetzt. Die Ausbeute an Dihydrocumarin liegt bei 60 %.

In EP A 0420532 wird ebenfalls die Herstellung von Dihydrocumarin ausgehend von 2-Oxocyclohexanpropionsäure in Gegenwart von Platin, Palladium, Nickel, Rhodium oder Iridium-Katalysator duch Dehydrierung und Cyclisierung beschrieben.

Das als Nebenprodukt anfallende Cumarin wird mittels eines Palladium-Katalysators, der vorher mit Luftsauerstoff aktiviert wird, bei 80 bis 160°C und 2 bar Wasserstoffdruck zu Dihydrocumarin in 87 % Ausbeute überführt.

Nach den bekannten Verfahren läßt sich Dihydrocumarin nur mit unbefriedigender Ausbeute herstellen. Die Reaktionsbedingungen wie hohe Temperaturen, hohe Drücke und Umgang mit Luftsauerstoff sind im Produktionsmaßstab nur mit hohem technischen Aufwand durchzuführen.

Aufgabe der vorliegenden Erfindung war es, Dihydrocumarin wirtschaftlich, in großer Reinheit und guter Ausbeute herzustellen.

Es wurde ein Verfahren zur Herstellung von Dihydrocumarin durch Hydrierung von Cumarin gefunden, das dadurch gekennzeichnet ist, dass Palladium ohne Sauerstoff-Aktivierung als Katalysator eingesetzt wird und Cumarin bei Temperaturen <80°C hydriert wird.

Das erfindungsgemäße Verfahren wird bevorzugt ohne zusätzliches Lösungsmittel durchgeführt.

Palladiumkatalysatoren sind an sich bekannt (Methoden der organischen Chemie / Houben Weyl, Band IV/1c, Reduktion Teil 1, Georg Thieme Verlag, Stuttgart, 1980, Seiten 15 bis 562, Handbook of Heterogeneous Catalysis, Vol. 1-5, Seiten 2123 bis 2447; VCH Weinheim; 1997).

Die Katalysatoren werden in der Regel auf einen Träger aufgebracht. Als Träger kommen beispielsweise Aktivkohle, Siliciumdioxid, Calciumcarbonat, oder Aluminiumoxid in Frage. Der bevorzugte Träger ist Aktivkohle.

Der Träger für das erfindungsgemäße Verfahren enthält im allgemeinen 1 bis 20 Gew.-%, bevorzugt 5 bis 10 Gew.-% Palladium bezogen auf den trockenen Gesamtkatalysator.

Für das erfindungsgemäße Verfahren kann der Katalysator im trockenen oder feuchten Zustand (bis zu 60 % Wasser) verwendet werden.

Für das erfindungsgemäße Verfahren beträgt das Gewichtsverhältnis des eingesetzten Katalysators zu Cumarin 0,0001 bis 0,1 zu 1, bevorzugt 0,005 bis 0,05 zu 1.

Die Reaktionstemperatur für das erfindungsgemäße Verfahren liegt bei 30 bis 80°C, bevorzugt bei 75°C bis 65°C.

Der Wasserstoffdruck liegt bei 1 bis 100 bar, bevorzugt bei 5 bis 10 bar.

Die Reaktionszeit beträgt 2 bis 100 Stunden, bevorzugt 5 bis 40 Stunden.

Das erfindungsgemäße Verfahren kann durch die folgende Reaktionsgleichung dargestellt werden:

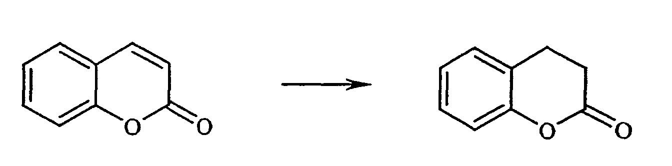

Das erfindungsgemäße Verfahren wird im allgemeinen wie folgt durchgeführt:

In einem Druckbehälter mit Rührer wird Cumarin und der Katalysator vorgelegt. Es wird bei der gewählten Reaktionstemperatur und Wasserstoffdruck hydriert. Das so erhaltene Dihydrocumarin wird nach Entfernen des Katalysators durch Filtration, Dekantieren oder Zentrifugation erhalten.

Nach dem erfindungsgemäßen Verfahren fällt Dihydrocumarin in einer Reinheit von 99,9 % und einer Ausbeute von 98 % an.

Dihydrocumarin ist ein Riechstoff mit süßem-krautigem Charakter, der an Heu erinnert. (S. Arctander, Perfume and Flavour Chemicals, No. 934, 1969).

### Beispiel

In einen 5 l Rührautoklaven mit Begasungsrührer werden 1500 g Cumarin und 6 g 5 gew.- %iges Palladium auf Aktivkohle feucht vorgelegt. Es wird 8 Stunden bei 75 bis 65°C und 5 bar hydriert. Nach Filtration werden 1492 g Dihydrocumarin mit einer Reinheit von 99,9 % erhalten. Das erhaltene Dihydrocumarin kann ohne Rückstand bei 140°C Sumpftemperatur und 3 mbar Vakuum destilliert werden. Die Ausbeute d. Th. beträgt 98 %.

## Patentansprüche

1. Verfahren zur Herstellung von Dihydrocumarin durch Hydrierung von Cumarin, **dadurch gekennzeichnet, dass** Palladium ohne Sauerstoff-Aktivierung als Katalysator eingesetzt wird und Cumarin bei Temperaturen <80°C hydriert wird.

2. Verfahren nach Anspruch 1, **dadurch gekennzeichnet, dass** das Gewichtsverhältnis des eingesetzten Katalysators zu Cumarin 0,0001 bis 0,1 zu 1 beträgt.

3. Verfahren nach den Ansprüchen 1 und 2, **dadurch gekennzeichnet, dass** die Reaktionstemperatur im Bereich von 30 bis 80°C liegt.

4. Verfahren nach den Ansprüchen 1 bis 3, **dadurch gekennzeichnet, dass** das Verfahren bei einem Wasserstoffdruck von 1 bis 100 bar durchgeführt wird.

## Claims

1. Process for the preparation of dihydrocoumarin by hydrogenation of coumarin, **characterised in that** palladium without oxygen activation is used as catalyst and coumarin is hydrogenated at temperatures of < 80 °C.

2. Process according to Claim 1, **characterised in that** the weight ratio of the catalyst used to coumarin is 0.0001 to 0.1 to 1.

3. Process according to Claims 1 and 2, **characterised in that** the reaction temperature is in the range from 30 to 80 °C.

4. Process according to Claims 1 to 3, **characterised in that** the process is carried out under a hydrogen pressure of 1 to 100 bar.

## Revendications

1. Procédé de préparation de dihydrocoumarine par hydrogénation de coumarine, **caractérisé en ce qu'**on utilise comme catalyseur du palladium sans activation par l'oxygène et qu'on hydrure la coumarine à des températures < 80°C.

2. Procédé selon la revendication 1, **caractérisé en ce que** le rapport en poids du catalyseur utilisé à la coumarine est de 0,0001 à 0,1.

3. Procédé selon les revendications 1 et 2, **caractérisé en ce que** la température réactionnelle est dans la plage de 30 à 80°C.

4. Procédé selon les revendications 1 à 3, **caractérisé en ce que** le procédé est réalisé à une pression d'hydrogène de 1 à 100 bar.
